# EUROPEAN PATENT SPECIFICATION

(11) **EP 1 034 261 B1**
(45) Date of publication and mention of the grant of the patent: **21.05.2008**
(21) Application number: 98965172.4
(22) Date of filing: 23.11.1998
(51) Int. Cl.: C12N 15/10, C12Q 1/68

(54) **METHOD OF PARALLEL SCREENING FOR INSERTION MUTANTS AND A KIT TO PERFORM THIS METHOD**
METHODE ZUM PARALLELEN AUFFINDEN VON INSERTIONSMUTANTEN UND REAGENTIEN UM DIES ZU BEWERKSTELLIGEN
PROCEDE DE CRIBLAGE EN PARALLELE DE MUTANTS D'INSERTION ET SON NECESSAIRE DE MISE EN OEUVRE

(30) Priority: 25.11.1997 EP 97203680
(43) Date of publication of application: 13.09.2000
(73) Proprietor: Oryzon Genomics, S.A., 08028 Barcelona (ES)
(72) Inventor: MAES, Tamara, E-08028 Barcelona (ES); GERATS, Tom, B-9000 Gent (BE)
(74) Representative: Barlocci, Anna
(86) International application number: PCT/EP1998/007551
(87) International publication number: WO 1999/027085

(56) References cited:
- WO-A-95/23875
- WO-A-96/17958
- R. KOES ET AL.: "Targeted gene inactivation in petunia by PCR-based selection of transposon insertion mutants" PROC. NATL. ACAD. SCI., vol. 92, August 1995, pages 8149-8153, XP002061408 NATL. ACAD. SCI.,WASHINGTON,DC,US; cited in the application
- R.R. ZWAAL ET AL.: "Target-selected gene inactivation in Caenorhabditis elegans by using a frozen transposon insertion mutant bank" PROC. NATL. ACAD. SCI., vol. 90, August 1993, pages 7431-7435, XP002087347 NATL. ACAD. SCI.,WASHINGTON,DC,US; cited in the application
- HENSEL M ET AL: "SIMULTANEOUS IDENTIFICATION OF BACTERIAL VIRULENCE GENES BY NEGATIVE SELECTION" SCIENCE, vol. 269, 21 July 1995, pages 400-403, XP000645478
- E. SOUER ET AL.: "A general method to isolate gene tagged by a high copy number transposable element" PLANT JOURNAL, vol. 7, no. 4, 1995, pages 677-685, XP002061409 BLACKWELL SCIENCE LTD, OXFORD, UK cited in the application
- SHALON D ET AL: "A DNA MICROARRAY SYSTEM FOR ANALYZING COMPLEX DNA SAMPLES USING TWO-COLOR FLUORESCENT PROBE HYBRIDIZATION" GENOME RESEARCH, vol. 6, no. 7, 1 July 1996, pages 639-645, XP000597095
- SCHENA M ET AL: "QUANTITATIVE MONITORING OF GENE EXPRESSION PATTERNS WITH A COMPLEMENTARY DNA MICROARRAY" SCIENCE, vol. 270, no. 5235, 20 October 1995, pages 467-470, XP000644675 cited in the application
- SHOEMAKER D D ET AL: "QUANTITATIVE PHENOTYPIC ANALYSIS OF YEAST DELETION MUTANTS USING A HIGHLY PARALLEL MOLECULAR BAR-CODING STRATEGY" NATURE GENETICS, vol. 14, no. 4, December 1996, pages 450-456, XP002043431 cited in the application
- VOS P ET AL: "AFLP: a new technique for DNA fingerprinting" NUCLEIC ACIDS RESEARCH, vol. 23, no. 21, 11 November 1995, pages 4407-4414, XP002086756
- D. VAN DEN BROECK ET AL.: "Transposon display identifies individual transposable elements in high copy number lines" PLANT JOURNAL, vol. 13, no. 1, January 1998, pages 121-129, XP002104299 Blackwell Science Ltd., Oxford, UK cited in the application
- T. MAES ET AL.: "Plant tagnology" TRENDS IN PLANT SCIENCE, vol. 4, no. 3, March 1999, pages 90-96, XP002104300 Elsevier Science, Amsterdam, NL

## Description

The current invention relates to a method of parallel and, as a consequence thereof, simultaneous screening for one or more gene insertion mutants in a population of any organism.

### Background of the invention

Gene disruption is a powerful tool to assign biological functions to the proteins that are encoded by, for example, the numerous uncharacterised open reading frames (ORF) resulting from genome projects or expressed sequence tags (EST) databases. To characterise the biological function of these ORFs efficiently, three problems must be addressed: (i) how to obtain a saturated population of mutants (ii) how to efficiently identify the genes that have been mutated in the different individuals or vice versa, how to find individuals mutated in a specific gene, and (iii) how to analyse their phenotypes.
Several strategies have been reported to disrupt specific genes or to simplify the identification of disrupted ORFs. In *Saccharomyces cerevisiae,* tagged mutants have been generated *en masse* by using random transposition of the endogenous Ty1 element (Smith *et al.,* 1995). Alternatively, a yeast library in *E.coli* was mutagenised with a Tn3 element (carrying lacZ for the monitoring of expression) followed by homologous recombination of the mutated yeast sequences into the yeast genome (Bums *et a*/*.,* 1994). Shoemaker *et al.* (1996) described an approach to perform a quantitative phenotypic assay of yeast deletion mutants, consisting of three steps: (i) replacement of each of the ∼6000 ORFs of *S. cerevisiae* with a unique 20bp tag and a kanamycin marker by homologous recombination, (ii) pooling of the deletion mutants and competitive growth under different selection conditions, (iii) amplification of the tags of the surviving strain and identification of their corresponding genes by hybridization of the isolated tags to a micro array displaying oligonucleotides complementary to the tags for all the yeast genes.
For other organisms, it is not yet possible or too laborious to obtain mutants through homologous recombination. Instead, large populations of individuals carrying random transposon insertions have been generated for *Drosophila melagonaster* (Ballinger and Benzer, 1989, Kaiser and Goodwin, 1990), *Caenorhabditis elegans* (Zwaal *et al.,* 1993), *Petunia hybrida* (Koes *et al.,* 1995) and Zea *mays* (Das and Martienssen, 1995). For *Arabidopsis thaliana,* large populations of T-DNA insertion mutants have been obtained through *Agrobacterium* mediated transformation (Bechtold *et al.,* 1993, Mc Kinney *et al.,* 1995, Mollier *et al.,* 1995). A PCR based approach is used to screen these populations for individuals harbouring insertions in genes of interest. By combining a gene-specific and an insertion-specific primer in the PCR screen, specific products are amplified only when the transposable element has inserted in that gene (Ballinger and Benzer, 1990, Kaiser and Goodwin, 1990). These populations are commonly organized in pools in a piramidal or 3D fashion, to allow fast screening and easy identification of the individuals carrying a mutant allele.
Using such an approach, the PCR screen has to be repeated for each individual ORF, or at the most, a limited set of gene-specific primers can be combined in the same screen (Mc Kinney *et al.,* 1995). This is a time consuming process and is extremely laborious to carry out.

The method according to the invention offers an attractive alternative to the existing strategy used to screen large populations for insertion mutants. The rationale of the previously described PCR based screening for insertion mutants is that whenever an insertion element has inserted in a gene, a product can be amplified in a PCR reaction using a gene-and an insertion-specific primer (Ballinger and Benzer, 1989; Kaiser and Goodwin, 1990).
The technique according to the invention reproducibly amplifies *all* the transposon flanking sequences in a pool of individuals, and subsequently identifies insertions by hybridization with a gene-specific probe. By eliminating the use of the gene-specific primer, it becomes possible to perform a serial screening for insertions in a large set of genes using a single set of PCR reactions. Alternatively, the flanking sequence pools can be used as probes to screen target genes.
This approach was tested using nested iPCR as a means to amplify the transposon *dTph1*-flanking sequences from the DNA pools of a 3D library of 1000 W137 *Petunia* individuals. To amplify *dTph1*-flanking sequences a tetracutter was used that does not cut within the element, the obtained DNA fragments were circularized, *dTph1* flanking sequences were amplified by iPCR using a set of internal primers, and a single primer based on the TIR for nested reamplification. The results indicate that a *dTph1* insertion in a specific gene of a specific plant can be detected against a background of about hundred wild type individuals, both in the serial and in the parallel screening method (see below). The variation in the signals produced for the positive control insertion in *PhAp2A* however indicate that the protocol had to be optimized to ensure that the *dTph1* flanking sequences of *all* the pool samples representing the population are sufficiently well amplified. The efficiency of the tagging system will influence the optimal size of the population to be screened. If n insertions per 1000 plants are found for a specific cDNA clone, 3n signals will arise. Direct identification of the candidate plant is only possible if n equals one. Somatic insertions into a gene will interfere and may lead to background hybridization. Stable insertion systems (for example T-DNA insertions) or systems with a controllable or low somatic insertion frequency will not suffer this problem.
The choice of the targets may also influence the efficiency of recovery of phenotypic mutants. If only cDNA targets are used, the percentage of intron and promotor amplified fragments that are recovered should statistically go down, and more knock-out mutants may be recovered, especially if the transposable element is small, harbours no transcription termination signals, and is unlikely to interfere with the gene function when it has inserted in an intron, like *dTph1* in *Petunia.*
The presented technique can be applied to the respective insertion mutagenesis system used for any organism. When the tetracutter recognizes a site within the insertion element, as it will in most cases, a set of two insertion specific primers must be used to amplify the flanking sequences. Instead of iPCR, the transposon flanking sequences can also be obtained by adaptor mediated PCR, as described by Van Den Broeck *et al.,* (1998) or by vectorette PCR (Riley *et al.,* 1990). The filter based screening, described here to illustrate the potential of the technique, could readily be extended to the microarray technology. In theory, microarrays can be produced displaying up to and over 20,000 targets, and hybridization of fluorescently labelled flanking sequences with those targets can be detected by means of confocal microscopy (Schena *et al.,* 1995). When (micro)arrays, orderly representing the whole genome of an organism are available, the method could be used to quickly catalogue the individual insertions and simultaneously assess the randomness of the insertions in a given population. Micro-arrays containing (c)DNA targets of an organism can also be used to screen for tagged genes in a closely related organism that is not in itself the subject of major sequencing efforts, if the stringency conditions used in the hybridization reactions are adapted. Such catalogues could also be helpful in identifying genes in map-based cloning strategies. After fine mapping of an interesting mutation, seeds of all the different insertion mutants in the region of interest could be retrieved from the insertion library. Mutant phenotypes in the progenies of the primary insertion mutants could then be compared to that of the originally mapped mutant. When the same or a comparable phenotype arises in such a family, the gene of interest has probably been tagged and thus been identified. Catalogues of insertion mutants can also be generated by isolation and sequencing all the insertion flanking sites found in a population. Using an efficient gene- and enhancer-trap transposon mutagenesis system, based on the maize elements Ac/Ds and the GUS reporter gene, a population of more than 10,000 single copy insertions mutants in *Arabidopsis* has been generated and a few hundred of these insertion alleles have been sequenced. The *dTph1* element in *Petunia* has a high copy number (150-200) in the line used for tagging. Every W137 individual harbours ten to twenty new germline insertions. Analysis of the new insertion sites would require cloning of the individual *dTph1* flanking sequences and selection of the 10% of clones that represent novel insertions. For high copy number tagging systems like *dTph1* in *Petunia, Mu* in maize and *Tc1* in *C.elegans,* the sequencing approach therefore seems too laborious. The current screening system according to the invention offers the opportunity to screen for insertion mutants in large populations of individuals. When used at its full potential, the efficiency of screening will be increased several orders of magnitude over that of the existing PCR based screening method.

So the first aspect of the current invention is a method of simultaneously screening for one or more gene insertion mutants in a population of any organism by :
a) preparing an insertion element mutant library originating from a defined population of an organism or cell line wherein said insertion(s) have to be detected,
b) amplifying the insertion element flanking sequences from said insertion element mutant library,
c1) either fixing the set of thus obtained nucleic acid amplification products representing said insertion element flanking sequences derived from said insertion element mutant library to a solid support as target for hybridization, or
c2) producing a set of labelled amplification products representing said insertion element flanking sequences derived from said insertion element mutant library to use as probe to hybridize to a solid support to which one or more nucleic acids have been fixed as target(s) for hybridisation.

The thus obtained nucleic acid amplification products in above captioned step b) can for example be obtained by iPCR using at least one primer or a set of primers based on the sequence of the insertion element or by Transposon Display PCR.

Above-mentioned iPCR is performed by
a) digesting the nucleic acid sequences of said insertion element mutant library with a restriction enzyme which optionally recognizes motifs of four nucleotides in the genomic DNA, or with a combination of restriction enzymes resulting in a collection of amplifyable fragments,
b) self ligation of the genomic fragments thus obtained and either
c1) amplification of insertion element flanking sequences using a set of internal primers or
c2) amplification of insertion element flanking sequences using a primer or a set of primers based on the terminal part of the insertion element.

The above described amplification products of step c1 can be re-amplified using at least one primer or a set of two nested primers based on the sequence of the insertion element.

The above-described amplification products in step b) can also be obtained by so-called transposon display amplification whereby said transposon display amplification is performed by
a) digesting the nucleic acid sequences of said insertion element mutant library with a first restriction enzyme that recognizes six conserved nucleotides in the insertion element and with a second restriction enzyme that recognizes a motif of four nucleotides in the genome generating at least one restriction fragment per insertion containing at least the hexacutter site, a part of the insertion element, and part of the insertion element flanking sequence,
b) ligation of a biotinylated adaptor to the hexacutter sites and a ligation of a second adaptor to the tetracutter sites of the restriction fragments generated in a),
c) selection of biotinylated restriction fragments using magnetic streptavidin beads,
d) amplification of insertion element flanking sequences using a primer based on the sequence of the biotinylated adaptor and on the insertion element sequence and a primer complementary to the second adaptor,
e) re-amplification of said insertion element flanking sequences using a nested primer based on the insertion element and a primer complementary to the second adaptor.

The solid support as described above in any of the methods can be a filter, micro-array, microchip containing nucleic acid sequences or a bead and the like, whereas the nucleic acid sequence is genomic DNA, cDNA, oligonucleotide sequence or PNA.

In the method according to the invention an insertion element mutant library may comprise 30 DNA samples from 100 plants each wherein the insertion element mutant library is built in a 3D array of 10 Block, 10 Row and 10 Column pools, each containing DNA of 100 plants characterised by the three coordinates B, R, C.

Depending on which method according to the current invention is used, Bfal or Msel and/or Munl are used as restriction enzyme.

The organism, having a genome wherein one or more gene insertions according to the invention has to be detected, can in principle be any organism including micro-organisms such as yeast, C.elegans, monocytelydoneous or dicotyledoneous plants such as preferably maize, but also Arabidopsis thaliana, Petunia and other flowering plant species and the like.

The set of insertion element flanking sequences can optionally be labelled with for instance fluorescein, however any suitable label known to a person skilled in the art can be used for this purpose.

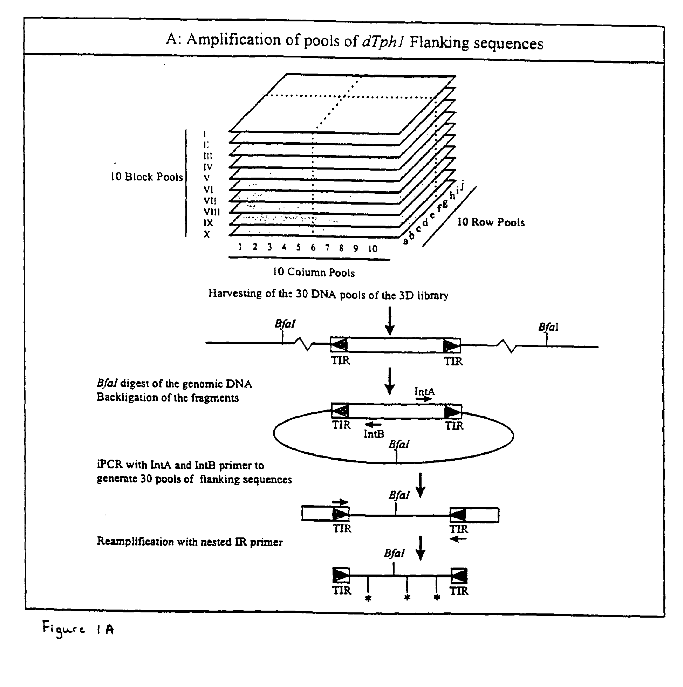

The system wherein the organised genomic DNA library according to the invention is present, can be, although not limited to, a so-called three dimensional system (3-D array as depicted in Figure 1A) and is clearly described in Koes et al., (1995) being herewith incorporated by reference.

The current invention is hereunder described in more detail for sake of clarity to get a better understanding of the invention.
In addition some definitions are hereunder given of what is meant by terms used in the current description.

### Definitions

-Restriction endonuclease or restriction enzyme: is an enzyme that recognizes a specific nucleotide sequence (target site) in a double-stranded DNA molecule, and will cleave both strands of the DNA molecule at every target site. The cleavage of the DNA molecule can appear at different positions on the two strands, resulting in the generation of single stranded DNA overhangs, which are specific for the restriction endonuclease.

-Restriction fragments: DNA molecules produced by digestion with a restriction enzyme. Any given genome will be digested by a particular restriction enzyme into a discrete set of restriction fragments.

-Insertion element refers to any nucleic acid sequence integrated in a genome. Insertion elements comprise, by way of example, but are not limited to: transposable elements, retrotransposons and T-DNA insertions.

-Insertion element flanking sequences are the nucleotide sequences flanking a given insertion element in the genome. Insertion flanking sequences can be amplified, by way of example, but not limited to, inverse PCR (iPCR), Transposon Display, Amplification of Insertion mutagenized sites (AIMS), vectorette PCR, or thermally asymmetric interlaced PCR (TAIL-PCR).

-The insertion element mutant library in this invention refers to a set of DNA samples isolated from and representing the population. The DNA pools are organized in a way that allows to refer to the individual or cell line in which an insertion element has inserted in a defined position in the genome, when an insertion is detected in a specific subset of DNA pools of the insertion element mutant library. By way of example, the insertion element mutant library representing a population of 1000 individuals may be obtained by giving each of the 1000 individuals three coordinates (B, R, C) with (1<=B<=10, 1<=R<=10, 1<=C<=10), and to isolate 30 DNA samples containing the DNA of the 100 individuals, in which each DNA sample is derived from the 100 individuals for which one of the three coordinates equaled a number between 1 and 10.

-Ligation is the joining of the phosphorylated 5' end of a nucleic acid to a 3' end of a nucleic acid. Ligation of double stranded DNA is mediated by the enzyme DNA ligase and involves the joining of the 5' and the 3' end of a DNA molecule to the 3' and 5' end a DNA molecule. The 5' end of a nucleic acid can be joined to a 3' end of a second nucleic acid in an intermolecular reaction, or it can be joined to its own 3' end in an intramolecular reaction which will be referred to as backligation or selfligation.

-Polymerase Chain Reaction (PCR): enzymatic reaction wherein DNA fragments are synthesized from a substrate DNA in vitro as described by Cetus in US Patent nrs 4,683,195; 4,683,202. Briefly, the reaction involves the use of two synthetic oligonucleotides / primers, which are complementary to nucleotide sequences in DNA molecules which are separated by a short distance of a few hundred to a few thousand base pairs, and the use of a thermostable DNA polymerase. After denaturing double-stranded DNA molecules, the primers will anneal to the complementary nucleotides in the template molecule at a specific annealing temperature. A suitable DNA polymerase then initiates the synthesis of a DNA strand complementary to the template molecule.The chain reaction continues for a variable number of cycles for instance a series of 10 to 30 cycles.

-DNA amplification: this term is used to denote the in vitro synthesis of double-stranded DNA molecules using for instance PCR as nucleic acid sequence amplification technique. The products of the PCR are called amplified DNA fragments.

-Primers: this term , in general, refers to a DNA strand which can prime DNA synthesis. DNA polymerase cannot synthesize DNA *de novo* without primers.

-An adaptor is a double stranded oligonucleotide that contains a single stranded DNA overhang that can be joined by ligation to a restriction fragment that contains a complementary DNA overhang.

-Nucleic acids comprise DNA, RNA, or synthetical analogues thereof. The genome of an organism consists of deoxyribonucleic acid (DNA). DNA is a double helix consisting of a two chains of nucleotides. Each chain consists of a strand of nucleotides linked to each other via phosphodiester bridges between the 5' hydroxylgroup of the sugar of one nucleotide linked to the 3' hydroxylgroup of the sugar of the adjacant nucleotide. As a consequence, a single stranded DNA molecule has an orientation defined by its 5' and its 3' end. DNA duplexes contain two single stranded DNA molecules joined together in the opposite orientation. Single stranded DNA consists of a combination of four different nucleotides, containing the bases: adenine (A), guanine (G), thymine (T) and cytosine (C). The DNA duplex is a result of the formation of hydrogen bridges between guanine and cytosine and between adenine and thymine residues located on the two different strands. Guanine and cytosine are complementary bases, as are thymine and adenine.
The information contained within the genome is transcribed into RNA. RNA is a single stranded chain of ribonucleic acid. RNA consists of four different nucleotides, containing the bases: adenine, guanine, cytosine and uracil. Uracil and adenine are complementary bases.

-Hybridization is the process of the binding of a nucleic acid to a second nucleic acid through the formation of hydrogen bridges between the bases of the nucleic acids. Hybridization of nucleic acids is obtained by interaction of two complementary nucleic acids at specific conditions of ionic strength, pH and temperature.

-Denaturation is the process of separation of two nucleic acid strands. Denaturation can be obtained by increasing the temperature or lowering the ionic strength of a double stranded nucleic acid solution.

-A single stranded nucleic acid is complementary to a second single stranded nucleic acid in case the two strands contain the complementary nucleotides in the opposite order. A nucleic acid can hybridize to a molecule which is its perfect complement, to a nucleic acid which comprises its perfect complement, or to a nucleic acid which is comprised in its perfect complement. Under tolerant hybridization conditions, a nucleic acid can also hybridize to non-perfect complements, provided the non-perfect complement has a sufficient amount of similarity with the perfect complement. Nucleic acids which are not identical but which exhibit a significant amount of similarity are called homologous.

-The term probe refers to a single stranded nucleic acid which is capable of binding to a second nucleic acid, which is called the target.
Typically, the probe is associated with a label to identify the target to which the probe binds.

-The term label refers to a molecular moiety capable of detection and may include, by way of example, radioactive isotopes, chemoluminiscense, fluorescence, dyes, etc. A label can also be a moiety which is capable of interacting with other molecules, which can be detected directly or indirectly, such as, by way of example, but not limited to, antigens which can be detected immunologically by means of antibodies, coupled to enzymes which can convert a substrate into a detectable molecule.

-in this invention, the term probe comprises the nucleic acid or the pool of nucleic acids which is hybridized to the target, the probe being a nucleic acid or a pool of nucleic acids in solution, and the target being a nucleic acid or a set of nucleic acids fixed to a solid support. Solid support refers to conventional supports comprising filters, membranes, silicate supports such as glass but also refers to more sophisticated supports like micro-arrays or microchips containing nucleic acid sequences.

-The term "population" comprises a group of discrete individuals or a group of discrete cell lines, a group containing at least one but typically 1000 individuals or cell lines.

### More detailed outline of the technique

To screen for insertion mutants in an efficient way, the plants are not analysed individually but organized for example in a 3D array of Block, Row and Column pools of 100 plants as described by Koes *et al.* (1995). In a population of 1000 plants, each individual is characterised by 3 coordinates (B_{1≤B≤10}, R_{1≤R≤10} ,C_{1≤C≤10}).
Ten Block, ten Row and ten Column pools of DNA are prepared of 100 plants each generating an insertion element mutant library consisting of 30 DNA samples.

### Screening for insertion mutants using iPCR and/or Transposon Display

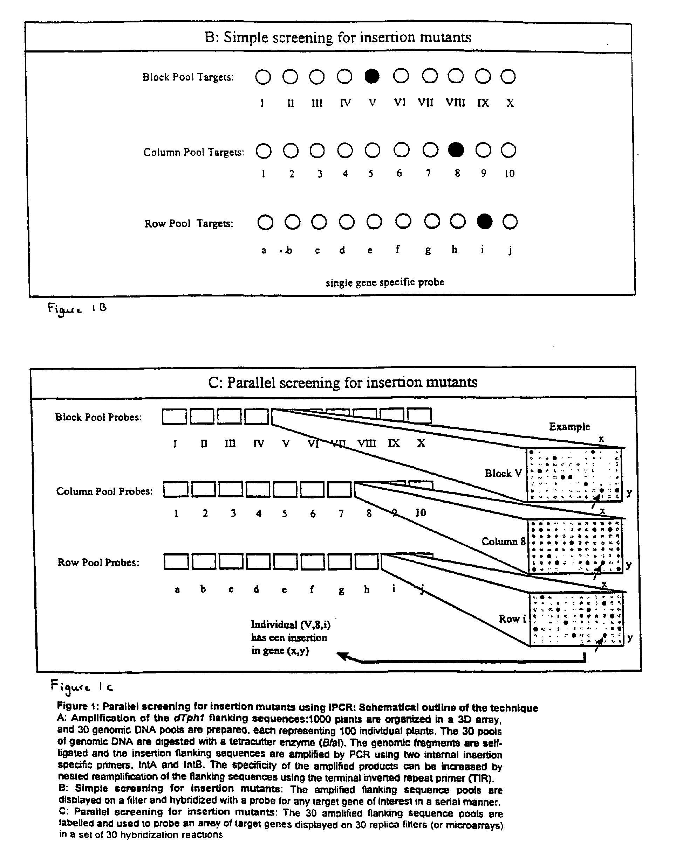

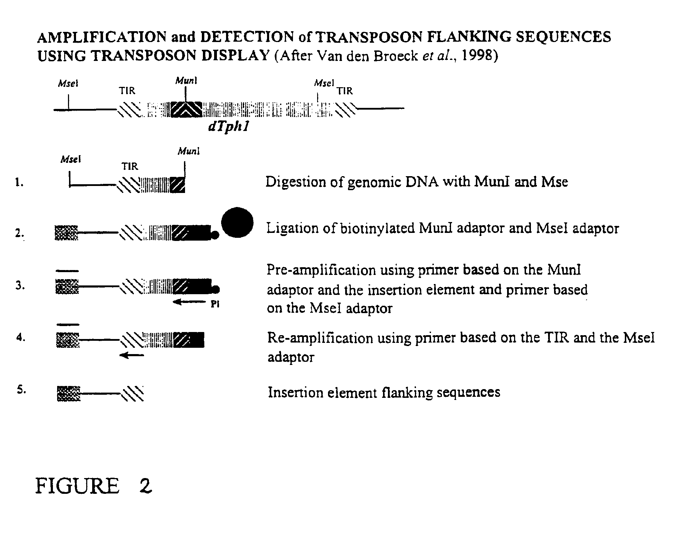

The insertion element flanking sequences in the different samples of the insertion element mutant library are recovered in four steps. Genomic DNA is digested using a tetracutter restriction enzyme. The obtained fragments are circularized by selfligation, amplified using a set of internal primers derived from the insertion element, and reamplified by nested PCR to ensure the specificity of the obtained products (Figure 1A).
Alternatively, the sequences flanking the insertion elements in a specific pool can be recovered using TRANSPOSON DISPLAY amplification. In this case, the DNA samples of the insertion elements mutant library are digested with a restriction enzyme recognizing a site of 6 bases (this restriction enzyme is referred to as the hexacutter) conserved in the insertion element and a restriction enzyme recognizing a site of 4 bases (this restriction enzyme is referred to as the tetracutter). Adaptors are ligated to the obtained fragments and those fragments containing the hexacutter site are selected. Insertion flanking sequences can be amplified in two steps. In the pre-amplification reaction a primer based on the hexacutter site adaptor and the insertion element is used in combination with a primer based on the tetracutter site adaptor to amplify a subset of PCR fragments. In the second step, these fragments are used as a template to amplify insertion specific fragments using a transposon specific primer and a primer based on the tetracutter site adaptor (Figure 2).
To screen for individuals carrying insertions in specific genes of interest, two approaches are possible.
In the first approach, the 30 amplified flanking sequence pools can be displayed on filters and hybridized with one target gene of interest at a time (Figure 1B). This method already offers an advantage over the existing gene-specific primer based methods, since the same PCR products can be used to screen many, at least 300-500, genes while upscaling is possible. In the second approach, a large set of targets is displayed on 30 replica filters, and hybridized with the labelled set of 30 amplified sequence pools (Figure 1 C). This approach becomes useful when more than 30 target genes are being screened for. One great advantage of this method is that *a priori* no sequence information of target genes is needed.

### Determining the detection limits

A reconstruction experiment was performed in which *Petunia* W137 genomic DNA was mixed with decreasing amounts of DNA of the heterozygous insertion mutant *PhAp2A* (V2025-6) and subjected to the iPCR protocol. The iPCR products were blotted on mini filters and hybridized to a range of dilutions of an *Ap2A* specific probe. At a probe concentration of 250pg/ml the hybridization signal of the 1/256 *PhAp2A*(V2025-6)/ W137 dilution could clearly be detected above the background signal. Since *Petunia hybrida* W137 individuals harbour around 150-200 *dTph1* elements , this means that a specific transposon flanking sequence was detected against a background of -50000 (256x200) copies. Consequently, specific insertions should be detectable in pools of 100 W137 *Petunia* plants. For other model systems, where less insertions are to be expected per individual, bigger pools could be screened.

### Construction of a 3D insertion flanking sequence library and screening for insertion mutants

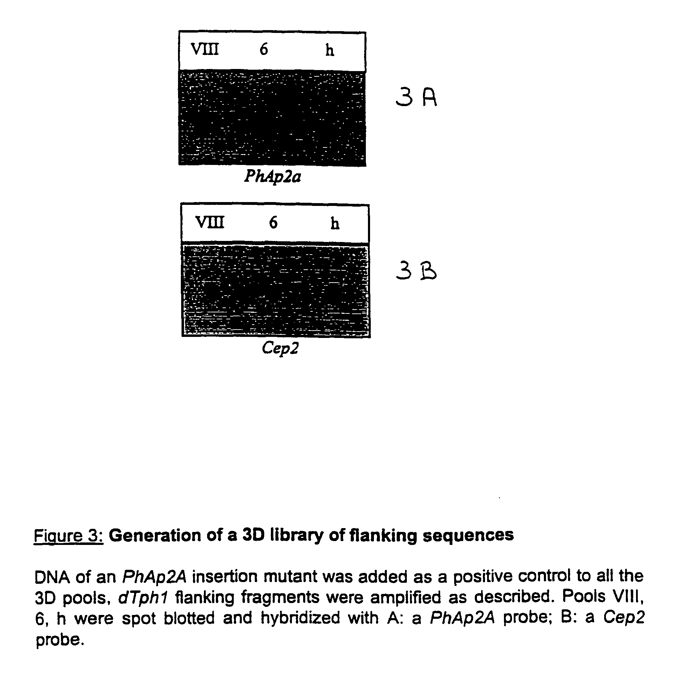

Thirty genomic DNA samples were prepared of pools of a hundred individuals each, representing a population of 1000 W137 plants. An individual plant carrying the PhAp2A(V2025-6) insertion allele was included in the population to serve as a positive control. The flanking sequences of the 30 DNA pools and an insertion mutant dilution series were generated using iPCR with the terminal inverted repeat primers of the transposon, with a set of internal transposon primers or combining the latter iPCR reaction with a nested reamplification with the terminal inverted repeat primers of the transposon (compare Fig.1A). The last approach clearly gave the best amplification on the pools.
For the technique to work, it is essential that all pools have efficiently amplified the flanking sequences of the *dTph1* elements. To control the general quality of the amplification reactions, we checked whether two *dTph1* flanking fragments, *Pete1* and *Pete2,* had been amplified equally in all pools. *Pete1* and *Pete2* were identified by Transposon Display (Van den Broeck *et al.,* 1998) and are inherited by all the individuals of the W137 population. This implies that they are present at a 200 fold higher concentration in the pools than a heterozygous insertion present in a single plant of the population. All pools were found positive for *Pete1* and *Pete2,* except R9 in the first series and R5 in the second series.
However, the flanking sequences of *dTph1* in Cep2 in plant (VIII, 6, h) of this population, previously identified by the classical 3D screening approach, did only show up as a positive in one of the three dimensions.
Therefore, a more sensitive positive control was developed by adding a 1/100 dilution of genomic DNA of a plant heterozygous for the PhAp2A(V2025-6) insertion allele to all of the pools of genomic DNA. After digestion and nested iPCR, the pool products were spot blotted and hybridized with a *PhAp2A* specific probe. The protocol was repeated until all pools proved positive for the *PhAp2A* insertion. After this qualitative improvement, the Cep2 insertion could be detected in the pools that we expected to be positive (Figure 3A and 3B).

### Parallel screening for insertion mutants

For parallel screening, the transposon flanking sequence pools were labelled and used as a probe to hybridize potentially tagged genes. A dilution series of *PhAp2A* and negative control target DNA (vector sequence) was spotted on small round filters and hybridized with a labelled Block III pool to determine the amount of DNA that needed to be displayed on the target filters to obtain a clear positive signal. A set of 30 replicas, each displaying 80 target genes, was made using a Beckman Biomek 2000 Laboratory Automated Workstation. Most of the displayed targets (73) were PCR amplified inserts from random picked cDNA clones from a carpel specific cDNA library. Among the seven targets with known sequences was *PhAp2A,* which was included as a constitutive positive control for the 30 samples. Using the classical 3D screening method, a *dTph1* insertion had been identified in *PhAp2C* in plant (V, 8, g), thus *PhAp2C* was used as a positive control representing a single insertion mutant appearing in the population.

The 30 pools of transposon flanking sequences obtained by nested iPCR were fluorescein labelled and hybridized to the 30 target gene replicas. Each target was displayed three times and formed a pattern which can be easily distinguished from spurious spots on the filter. The control *PhAp2A* signal can be detected as a three dot diagonal at position (2, E) on each filter using probes made of the insertion element flanking sequences which had been amplified nicely as determined by the quality control assay described under construction of a 3D insertion flanking sequence library. A *dTph1* insertion in *PhAp2C* was detected in the correct position at the diagonal of position (4, E) of the filters hybridized with the probes made of the insertion element flanking sequences which passed the quality control assay, and which were derived from the insertion element mutant library samples which contained the DNA of the individual plant that contained a *dTph1* insertion in *PhAp2C.*

### Examples

### Method for determination of detection limits

Genomic DNA of the heterozygous *PhAp2A* insertion mutant was diluted with increasing amounts of wild type DNA (ranging from 1/1 to 1/256 insertion mutant/ wild type DNA). 10µg of each DNA mix was digested in 100µl 1x New England Biolabs buffer 4 with the tetracutter enzyme Bfal, which doesn't cut within the 284bp *dTph1* element. After complete digestion, the enzyme was heat inactivated and the mixture was phenol:chloroform extracted, precipitated and dissolved in dd H₂O. 2 µg of DNA was ligated ON at 14 °C in 400µl 1xT4 ligation buffer in the presence of 2.5 units of T4 DNA ligase. The ligation mixture was extracted with phenol:chloroform, with chloroform and precipitated in the presence of 20µg of calf thymus tRNA, washed and dried. The vacuum dried pellet was resuspended in 30µl of dd H₂O. 5µl of the self-ligated fragments were used in the iPCR reaction with the outward transposon inverted repeat primer (TIR), consisting of 35 cycles of 1min denaturation at 95°C, 1 min annealing at 55°C, and 1.5min polymerisation at 72°C. The PCR reactions were also performed on equivalent amounts of undigested W137 and insertion mutant DNA. The reaction products were spotted in 8-plicate on individual Hybond N⁺ filters that had been cut small enough to fit into 96 well plates, denatured, neutralised and baked as prescribed by the manufacturer. The filters were prehybridised in 50µl of hybridization mix (as prescribed by Amersham). 50ng of a 900bp *SpeI*/*EcoRI Ap2A* fragment was ³²P labelled with a Megaprime kit in a 50µl reaction with 5µl α-dCTP (incorporation ~50%). Half of the denatured probe was diluted to 1200µl with hybridization mix, 600µl was divided in 12 aliquots of 50µl, that were added to the prehybridization mix, 600µl was used for seven consecutive two-fold dilutions. Thus, the concentration of the probe in the first series of 12 hybridizations was ∼1ng/100µl, decreasing each time by a factor of two for the next seven dilutions. Washes were performed at 60°C, 2x in 2xSSC, 0.1%SDS and 4x in 0.5xSSC, 0.1%SDS for 10 min each. Hybridized filters were exposed to a Phosphor Image cassette and scanned using a Beckman Phosphor Imager.

### Method for the construction of a 3D insertion flanking sequence library including quality control

1000 W137 plants were organized in a 3D array of 10 blocks, 10 rows and 10 columns, as described by Koes et al. (1995). DNA was extracted from the leaves of the 30 pools using a CTAB extraction method. DNA extracted from a heterozygous *Ap2A* insertion mutant was added to each pool at a ratio of 1:100 as a positive control. 10µg of each of the 30 DNA samples was digested at 37°C for 6h by 10 units of Biolabs *Bfa*l restriction enzyme in 1x NEB4 buffer. The restriction reactions were extracted with phenol, phenol:chloroform and chloroform. 1µl of 10mg/ml tRNA was added, the samples were precipitated with NaOAc and EtOH, washed with 70% EtOH and dissolved in 100µl H2O. 20µl of this mix (2µg) was ligated overnight at 16°C by 6 units of T4 DNA ligase (Pharmacia) in 400µl of ligation buffer (10mM MgC12, 30mM Tris.Cl pH7.8, 10mM DTT, 0.5mM ATP). The ligation mixture was extracted with phenol, phenol:chloroform and chloroform, precipitated as above and dissolved in 30µl H2O. 5µl of this solution was used as template in a first amplification reaction performed with two internal transposon specific primers (IntA: 5'-GGGAATTCTTGAACGAGTTGTCCTC-3' and IntB: 5'-GGGAATTCAGTGTAAATTTTGCGC-3', see also Souer *et al.,* 1995), for 35 cycles of 30s at 94°C, 30s at 55° and 1min30s at 72°C in 50µl 1x Perkin Elmer with 30pmol of each primer, 200nM dNTPs, 4mM MgC12 and 1 unit Perkin Elmer Taq polymerase. 3µl of the resulting transposon flanking sequences were reamplified with a primer based on the terminal inverted repeat of the transposon (5'-GGGAATTCGCTCCGCCCCTG-3').

### Quality control of the amplified flanking sequence pools

0.25µl of the PCR products were spot blotted on a Hybond N⁺ membrane and hybridized with an α - ³²P dCTP *Ap2A* specific probe to see whether the control insertion mutant could be detected. Washes and detection were as in the reconstruction experiment.

### Method for the parallel screening of insertion mutants

20µl aliquots of target DNA solution (100ng/µl) were distributed in V bottom shaped microtiter plates and spotted on HybondN⁺ filters using a Beckman Biomek 2000 Laboratory Automation Workstation. Each replica had the size of 1/3 96 well microtiterplate. On each of the 4x8 positions (from 1 to 4 and from A to H), a matrix of 3 targets was displayed as follows:

| | | |
|---|---|---|
| a | b | c |
| a | c | b |
| c | a | b |

In this way, 4x8x3=96 targets can be displayed per replica (we have used a 4x7x3=84 target array). The filters were treated for hybridization as described by Amersham. 5-10µl of the amplified flanking sequence pools were labelled with fluorescein using the Gene Images random prime labelling module (Amersham). A small amount of IR primer was added in the labelling reaction to ensure that small fragments would be labelled. The replica filters were hybridized in 5ml hybridization buffer and washed according to the Amersham protocol. The hybridizing signals were visualised using the Gene Images Detection Module (Amersham) after exposure to Fuji X-RAY films.

### SCREENING FOR INSERTION MUTANTS USING TRANSPOSON DISPLAY

### Determining the detection limits

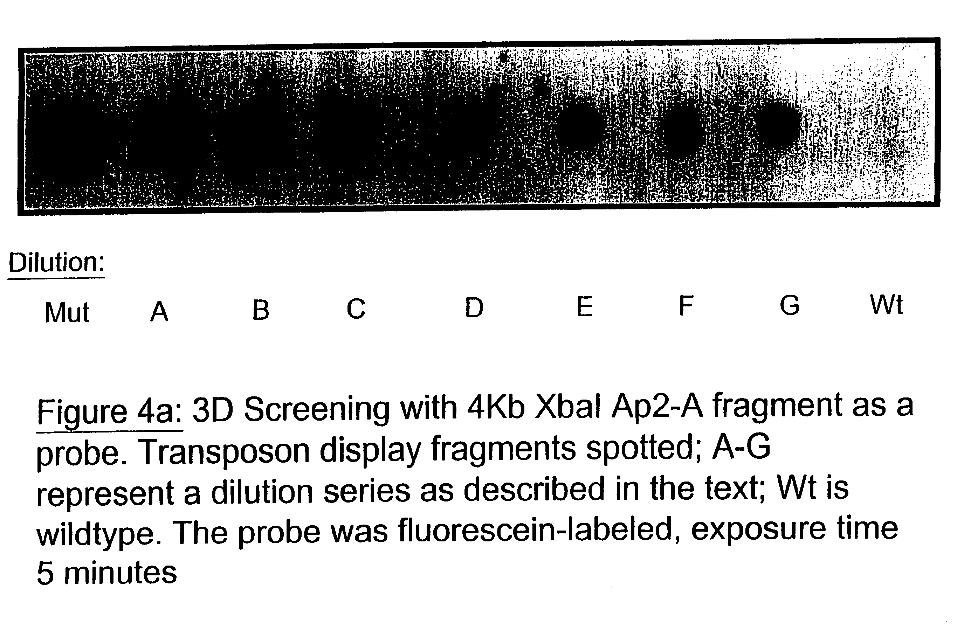

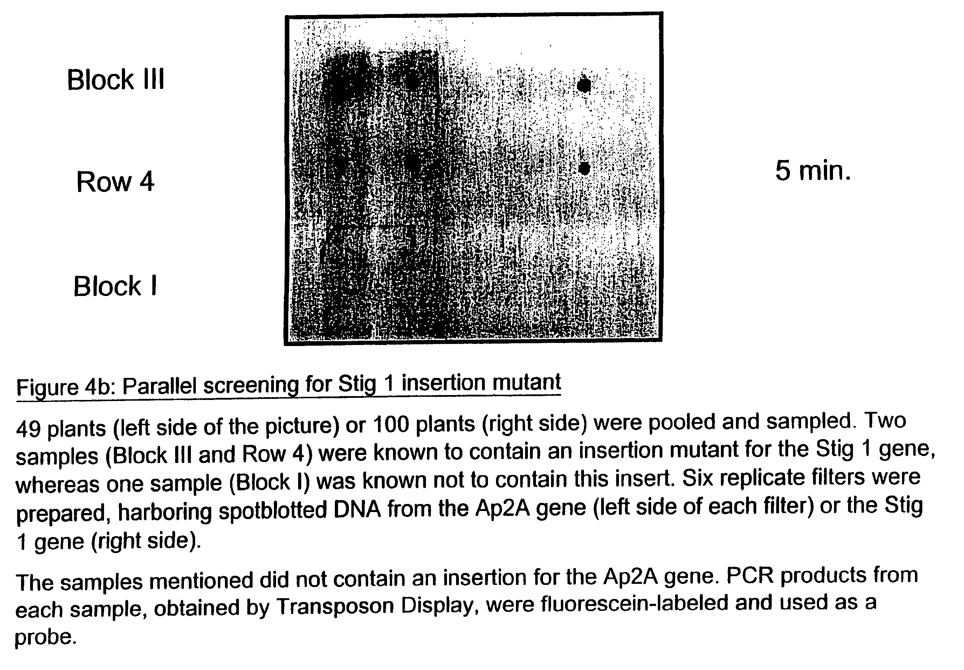

A reconstruction experiment was performed in which Petunia W137 genomic DNA was mixed with decreasing amounts of DNA of an insertion mutant containing a *dTph1* insertion in Ap2A (A=1/2, B=1/4, C= 1/8, D=1/16, E= 1/32, F=1/64, G=1/128), and subjected to the transposon display amplification protocol, which was performed with unlabeled PCR primers. The Transposon Display amplification products were spot blotted on filters and hybridized with a labeled *Ap2A* specific probe. The amplified *dTph1* flanking fragment containing part of the *Ap2A* gene could readily be detected in the 1/ 128 dilution of the mutant DNA with the wild type W137 DNA (Figure 4A).

### Construction of a 3D library of insertion flanking sequences and screening for insertion mutants.

A second reconstruction experiment was performed in which the genomic DNA of a pool of respectively 4 (2x2), 9 (3x3), 16 (4x4), 25 (5x5), 36 (6x6), 49 (7x7), 64 (8x8), 81 (9x9) and 100 (10x 10) different individuals, was isolated. In each pool, 1 of the individuals was subsequently replaced by an individual containing a *dTph1* insertion in the *Ap2A* gene and DNA's were extracted.
Transposon Display amplification was performed on each of the pools using unlabelled primers and the resulting Transposon Display amplification products were spot blotted on filters and hybridized with an *Ap2A* specific probe. The amplified *dTph1* flanking fragment containing part of the *Ap2A* gene could readily be detected as a positive signal in each of the pools harboring the Ap2A insertion. In control pools not containing DNA from the insertion mutant, no such signal was observed. The *dTph1* flanking sequences could then be amplified from all 30 pools of a population of 1000 organized in Block, Rows and Columns, spotted on a filter and used to screen for insertions in any gene of interest.

### Parallel screening for insertion mutants

In order to screen for insertions in many genes simultaneously, the amplified Transposon Display fragments are labelled and used as a probe to hybridize a filter containing a set of target genes.
Both Transposon Display fragments obtained from the amplification of *dTph1* flanking fragments from the dilution serie F and G, described above, and those obtained from the 2x2, 6x6, 8x8 and 10x10 pools containing an individual with a *dTph1* insertion in *Ap2A,* mentioned above, were separately labeled and used to hybridize filters displaying a fragment of the Ap2A gene. All probes obtained by Transposon Display PCR on the pools containing the Ap2A insertion, were able to detect the *Ap2A* gene on the filters.
The dTph1 flanking sequences can be amplified from all 30 pools of a population of 1000 organized in Block, Row and Columns, labeled, and used as probes to hybridize filters containing multiple gene targets. An insertion in gene *Stig1,* was indentified in the correct pools of Transposon Display fragments obtained from such a population (FIGURE 5B).

### METHODS TO SCREEN FOR INSERTION MUTANTS USING TRANSPOSON DISPLAY

Transposon Display was performed as described by Van den Broeck *et al.,* 1998, with the following modifications:
The streptavidin beads were suspended in 50 instead of 200µl T01E buffer, the selective pre-amplification reactions were performed with 5µl of the above 50µl in a reaction volume of 50µl using the *Mun*I-ACAC primer and an Msel primer, using however a touch down PCR profile as described in the hot PCR. The pre-amplification products were diluted 10 times and re-amplified using 5µl of the diluted pre-amplification product, the IR primer and the Msel primer, in a reaction volume of 50µl but without labeling the IR primer, resulting in the final product.

To perform simple screening for insertion mutants, the 50µl of final product was dried, resuspended in 5µl water, denatured and spotted on HybondN+, and hybridized with a denatured gene specific probe labeled using the CDP-star kit of Amersham, in a buffer containing 5xSSC, 60°C, and blots were washed at 60°C at a stringency of 0.5x SSC.

To perform parallel screening for insertion mutants, 2µl of the 50µl of the final product was labelled using a linear PCR using 1,5µl IR primer, 10µl nucleotide mix of the CDP-star kit of Amersham, 5µl 10xPCR buffer and 0,2µl Taq polymerase in a volume of 50µl, using a PCR profile of 30 cycles of 94°C/30s, 56°C/30s, 72°C/60s.
Hybridizations of filters with target genes were performed with these probes in a buffer containing 5xSSC at 60°C, and blots were washed at 60°C at a stringency of 0.5xSSC.

### References

Ballinger, D.G., and Benzer, S. (1989). Targeted gene mutations in Drosophila. PNAS 86, 9402-9406
Bechtold N. , Ellis, J., Pelletier, G. (1993). In planta Agrobacterium mediated gene transfer by infiltration of adult Arabidopsis thaliana plants. C. R. Acad. Sci. Paris, Sciences de la vie/Life Sciences 316, 1194-1199.
Burns, N., Grimwade, B., Ross-Macdonald, P.B., Choi, E-Y., Finberg, K., Roeder, G.S. and Snyder, M. (1994). Large-scale analysis of gene expression, protein localization, and gene disruption in Saccharomyces cerivisiae. Genes and Dev. 8, 1087-1105.
Das, L. and Martienssen, R. (1995). Site-Selected Transposon Mutagenesis at the hcf106 Locus in Maize. Plant Cell 7(3), 287-294
Gerats,A.G.M.,Beld,M., H u its, H., Prescott,A. (1989). Gene tagging in Petunia hybrida using homologous and heterologous transposable elements. Dev.Gen. 10, 561-568
Kaiser, K., and Goodwin, S.F. (1990). "Site-selected" mutagenesis of Drosophila. PNAS 87,1686-1690.
Koes, R., Souer, E., Van Houwelingen, A., Mur, L., Spelt, C., Quattrocchio, F., Wing, J., Oppedijk, B., Ahmed, S., Maes, T., Gerats, T., Hoogeveen, P., Meesters, M., Kloos, D., Mol, J.N.M. (1995). Targeted gene inactivation in petunia by PCR-based selection of transposon insertion mutants. PNAS 92(18), 8149-8153
McKinney, E.C., Aali, N., Traut, A., Feldmann, K. A., Belostotsky, D.A., McDowell, J.M., Meagher, R. (1995). Sequence-based identification of T-DNA insertion mutations in Arabidopsis: Actin mutants act2-1 and act4-1. Plant Journal 8(4), 613-622
Mollier, P., Montoro, P., Delarue, M., Bechtold, N., Bellini,C., Pelletier, G. (1995). Promoterless gusA expression in a large number of Arabidopsis thaliana transformants obtained by the in planta infiltration method. C.R.Acad.Sci. Paris, Sciences de la vie/Life sciences 318, 465-474.
Riley, J., Butler, R., Ogilvie, D., Finniear, R., Jenner, R., Powell, S., Anand, S., Smith, S., and Markham, A.F. (1990). A novel, rapid method for the isolation of terminal sequences from yeast artificial chromosome (YAC) clones. NAR 18, 2887-2890.
Schena, M., Shalon, D., Davis, R.W. and Brown, P.O. (1995). Quantative monitoring og gene expression patterns with a complementary DNA microarray. Science 270, 467-470.
Shoemaker, D.D., Lashkari, D.A., Morris, D., Mittmann, M. and Davis, R.W. (1996). Quantitative phenotypic analysis of yeast deletion mutants using a highly parallel molecular bar-coding strategy. Nature Genetics 14, 450-456.
Smith, V., Botstein, D. and Brown, P.O. (1995). Genetic footprinting: A genomic strategy for determining a gene's function given its sequence. PNAS 92, 6479-6483.
Souer, E., Quattrochio, F., de Vetten, N., Mol, J. and Koes, R. (1995). A general method to isolate genes tagged by a high copy number transposable element. Plant Journal 7(4): 677-685.
Van den Broeck, D., Maes, T., Sauer, M., Zethof, J., Van Montagu, M. and Gerats, T. (1998) Transposon Display identifies individual transposable elements in high copynumber lines. Plant Journal 13(1):121-129.
Zwaal, R.R., Broecks, A., Van Meurs, J., Groenen, J.T.M., Plasterk, R.H.A. (1993). Target-selected gene inactivation in Caenorhabditis elegans by using a frozen transposon insertion mutant bank. PNAS 90(16): 7431-7435

## Claims

1. Method of simultaneous screening for one or more gene insertion mutants in a population of any organism or cell line derived thereof by:
a) preparing an insertion element mutant library originating from a defined population of an organism or cell line wherein said insertion(s) have to be detected,
b) amplifying the insertion element flanking sequences from said insertion element mutant library,
c1) either fixing the set of thus obtained nucleic acid amplification products representing said insertion element flanking sequences derived from said insertion element mutant library to a solid support as target for hybridization, or
c2) producing a set of labelled amplification products representing said insertion element flanking sequences derived from said insertion element mutant library to use as probe to hybridize to a solid support to which one or more nucleic acids have been fixed as target(s) for hybridisation.

2. Method according to claim 1 wherein the thus obtained nucleic acid amplification products in step b) are obtained by iPCR using at least one primer or a set of primers based on the sequence of the insertion element.

3. Method according to claim 2 wherein the iPCR is performed by
a) digesting the nucleic acid sequences of said insertion element mutant library with a restriction enzyme which optionally recognizes motifs of four nucleotides in the genomic DNA, or with a combination of restriction enzymes resulting in a collection of amplifyable fragments,
b) self ligation of the genomic fragments thus obtained and either
c1) amplification of insertion element flanking sequences using a set of internal primers or
c2) amplification of insertion element flanking sequences using a (set of) primers based on the terminal part of the insertion element.

4. Method according to claim 3 wherein the amplification products of step c1 are re-amplified using at least one primer or a set of two nested primers based on the sequence of the insertion element.

5. Method according to claim 1 wherein the amplification products in step b) are obtained by transposon display amplification, which is performed by
a) digesting the nucleic acid sequences of said insertion element mutant library with a first restriction enzyme that recognizes six conserved nucleotides in the insertion element and with a second restriction enzyme that recognizes a motif of four nucleotides in the genome generating at least one restriction fragment per insertion containing at least the hexacutter site, a part of the insertion element, and part of the insertion element flanking sequence,
b) ligation of a biotinylated adaptor to the hexacutter sites and a ligation of a second adaptor to the tetracutter sites of the restriction fragments generated in a),
c) selection of biotinylated restriction fragments using magnetic streptavidin beads,
d) amplification of insertion element flanking sequences using a primer based on the sequence of the biotinylated adaptor and on the insertion element sequence and a primer complementary to the second adaptor,
e) re-amptification of said insertion element flanking sequences using a nested primer based on the insertion element and a primer complementary to the second adaptor.

6. Method according to any of the preceeding claims wherein the solid support is a filter, micro-array or chip containing nucleic acid sequences.

7. Method according to any of the preceeding claims wherein the nucleic acid sequence is genomic DNA or cDNA.

8. Method according to any of the preceeding claims werein the insertion element mutant library comprises of 30 DNA samples from 100 plants each.

9. Method according to claim 8 wherein the insertion element mutant library is built in a 3D array of 10 Block, 10 Row and 10 Column pool each containing DNA of 100 plants **characterised by** the three coordinates B, R, C.

10. Method according to any of the claims 1-4 wherein Bfal is used as restriction enzyme.

11. Method according to claim 5 wherein Msel and/or Munl are used as restriction enzyme.

## Patentansprüche

1. Methode zur simultanen Untersuchung von einem oder mehreren Insertionsmutanten von Genen in einer Population von irgendeinem Organismus oder einer davon abgeleiteten Zellenlinie:
a) Vorbereitung einer Insertionsmutanten-Bibliothek von einer definierten Population eines Organismus oder einer Zellenlinie, wobei die besagte(n) Insertion(en) ermittelt werden müssen,
b) Amplifikation der Flankensequenzen der Insertionselemente aus besagter Insertionsmutanten-Bibliothek,
c1) entweder durch Fixieren der so erhaltenen Amplifikationsprodukte der Nukleinsäure, wobei die besagten Flankensequenzen des Insertionselements dargestellt sind, die von der besagten Insertionsmutanten-Bibliothek abgeleitet sind für einen festen Trägerstoff, der für die Hybridisierung bestimmt ist, oder
c2) Produktion eines Satzes von benannten Amplifikationsprodukten, die besagte Flankensequenzen des Insertionselements darstellen, die von besagter Insertionsmutanten-Bibliothek abgeleitet sind, um diese als Probe zu benutzen zur Hybridisierung eines festen Trägerstoffs, auf welchen eine oder mehrere Nukleinsäuren zur Hybridisierung fixiert wurden.

2. Verfahren nach Anspruch 1, bei dem die Amplifikationsprodukte der so erhaltenen Nukleinsäure in einer Phase b) durch PCR erhalten werden unter der Verwendung von mindestens einem Primer oder einem Satz von Primern auf der Basis der Sequenz des Insertionselements.

3. Verfahren nach Anspruch 2 bei dem das PCR durchgeführt wird, durch
a) Aufschliessung der Nukleinsäurensequenzen von besagter Insertionsmutanten-Bibliothek mit einem Restriktionsenzym, das optional Motive von vier Nukleotiden in der genomischen DNA erkennt, oder mit einer Kombination von Restriktionsenzymen, was zu einer Sammlung von erweiterbaren Fragmenten führt,
b) Selbstbindung der so erhaltenen genomischen Fragmente und sowohl eine
c1) Amplifikation der Flankensequenzen der Insertionselemente unter Verwendung eines Satzes von internen Primern als auch
c2) Amplifikation der Flankensequenzen des Insertionselements unter Verwendung eines (Satzes von) Primern auf der Basis des Endteils des Insertionselements.

4. Verfahren nach Anspruch 3, bei dem die Amplifikationsprodukte der Phase c1 nochmals verstärkt werden unter der Verwendung von mindestens einem Primer oder von einem Satz von zwei geschachtelten Primern auf der Basis der Sequenz des Insertionselements.

5. Verfahren nach Anspruch 1, bei dem die Amplifikationsprodukte in der Phase b) durch eine Amplifikation der Visualisierung des Transposons erhalten werden, die wie folgt durchgeführt wird:
a) Aufschliessung der Nukleinsäurensequenzen von besagter Insertionsmutanten-Bibliothek mit einem ersten Restriktionsenzym, das sechs im Insertionselement konservierte Nukleotide erkennt und mit einem zweiten Restriktionsenzym, das ein Motiv von vier Nukleotiden im Genom erkennt, indem mindestens ein Restriktionsfragment pro Insertion erzeugt wird, das mindestens die Lage des Hexacutters, einen Teil des Insertionselements, und einen Teil der Flankensequenz der Insertionselemente enthält,
b) Bindung eines biotinylierten Adapters an die Lagen des Hexacutters und eine Bindung eines zweiten Adapters an die Lagen des Tetracutters der in a) erzeugten Restriktionsfragmente,
c) Auswahl von biotinylierten Restriktionsfragmenten unter Verwendung von magnetischen Streptavidin-Kügelchen,
d) Amplifikation der Flankensequenzen von Insertionselementen unter Verwendung eines Primers auf der Grundlage der Sequenz des biotinylierten Adapters und der Sequenz von Insertionselementen und ein komplementärer Primer zum zweiten Adapter,
e) Nochmalige Amplifikation der Flankensequenzen von besagten Insertionselementen unter Verwendung eines verschachtelten Primers auf der Grundlage des Insertionselements und ein komplementärer Primer zum zweiten Adapter.

6. Verfahren nach einem der vorhergehenden Ansprüche, bei dem der feste Träger ein Filter, Mikroarray oder Chip ist, das Nukleinsäurensequenzen enthält.

7. Verfahren nach einem der vorhergehenden Ansprüche, bei dem die Nukleinsäurensequenz eine genomische DNA oder cDNA ist.

8. Verfahren nach einem der vorhergehenden Ansprüche, bei dem die Insertionsmutanten-Bibliothek aus 30 DNA Proben von je 100 Pflanzen besteht.

9. Verfahren nach Anspruch 8, bei dem die Insertionsmutanten-Bibliothek in einem 3D-Array von 10 Blocks, 10 Reihen und 10 Säulenpools aufgebaut ist, je enthaltend eine DNA von 100 Pflanzen, **gekennzeichnet durch** die drei Koordinate B, R, C.

10. Verfahren gemäß einem der Ansprüche 1 - 4, worin Bfal als Restriktionsenzym benutzt wird.

11. Verfahren nach Anspruch 5, worin Msel und/oder MunI als Restriktionsenzym verwendet werden.

## Revendications

1. Méthode de filtrage simultané pour un ou plusieurs mutants d'insertion d'un gène dans une population de n'importe quel organisme ou lignée cellulaire dérivée de celui-ci par:
a) préparation de bibliothèque mutante d'un élément d'insertion provenant d'une population définie d'un organisme ou lignée cellulaire où ladite(lesdites) insertion(s) doit être détectée,
b) amplification des régions flanquantes d'un élément d'insertion de ladite bibliothèque mutante d'un élément d'insertion,
c1) soit par fixation de l'ensemble des produits d'amplification d'acide nucléique obtenus de cette manière et représentant lesdites régions flanquantes d'un élément d'insertion dérivées de ladite bibliothèque mutante d'un élément d'insertion pour un support solide destiné à l'hybridation, ou
c2) production d'un ensemble de produits d'amplification marqués représentant lesdites régions flanquantes d'un élément d'insertion dérivées de ladite bibliothèque mutante d'un élément d'insertion à utiliser sous forme de sonde pour hybrider un support solide auquel un ou plusieurs acides nucléiques ont été fixés prévus pour l'hybridation.

2. Méthode selon la revendication 1 où les produits d'amplification d'acide nucléique obtenus de cette manière lors d'une phase b) sont obtenus par PCR en utilisant au moins une amorce ou un ensemble d'amorces en base à la région de l'élément d'insertion.

3. Méthode selon la revendication 2 où la PCR est réalisée par
a) digestion des séquences d'acide nucléique de ladite bibliothèque mutante d'un élément d'insertion avec une enzyme de restriction qui reconnaît optionnellement des motifs de quatre nucléotides dans l'ADN génomique, ou avec une combinaison des enzymes de restriction produisant une collection de fragments amplifiables,
b) autoligature des fragments génomiques obtenus de cette manière et soit
c1) amplification des régions flanquantes d'un élément d'insertion en utilisant un ensemble d'amorces internes ou
c2) amplification des régions flanquantes d'un élément d'insertion en utilisant un (ensemble d') amorces ayant pour base la partie terminale de l'élément d'insertion.

4. Méthode selon la revendication 3 où les produits d'amplification de la phase c1 sont réamplifiés en utilisant au moins une amorce ou un ensemble de deux amorces emboîtées ayant pour base la région de l'élément d'insertion.

5. Méthode selon la revendication 1 où les produits d'amplification de la phase b) sont obtenus par amplification de visualisation du transposon, qui est réalisée par
a) digestion des séquences d'acide nucléique de ladite bibliothèque mutante d'un élément d'insertion avec une première enzyme de restriction qui reconnaît six nucléotides conservés dans l'élément d'insertion et avec une deuxième enzyme de restriction qui reconnaît un motif de quatre nucléotides dans le génome générant au moins un fragment de restriction par insertion contenant au moins le site hexacutter, une partie de l'élément d'insertion, et une partie de la région flanquante d'un élément d'insertion,
b) ligature d'un adaptateur biotinilaté avec les sites hexacutter et une ligature d'un deuxième adaptateur avec les sites tetracutter des fragments de restriction produits dans a),
c) sélection des fragments de restriction biotinilatés en utilisant des gouttes de streptavidine magnétiques,
d) amplification des régions flanquantes d'un élément d'insertion en utilisant une amorce en base à la séquence de l'adaptateur biotinilaté et à la région de l'élément d'insertion et une amorce complémentaire du deuxième adaptateur,
e) réamplification desdites régions flanquantes d'un élément d'insertion en utilisant une amorce emboîtée en base à l'élément d'insertion et une amorce complémentaire du deuxième adaptateur.

6. Méthode selon l'une quelconque des revendications précédentes où le support solide est un filtre, microréseau ou puce d'ADN contenant des séquences d'acide nucléique.

7. Méthode selon l'une quelconque des revendications précédentes où la séquence d'acide nucléique est ADN ou ADNc génomiques.

8. Méthode selon l'une quelconque des revendications précédentes où la bibliothèque mutante d'un élément d'insertion comprend 30 échantillons d'ADN de 100 installations chacun.

9. Méthode selon la revendication 9 où la bibliothèque mutante d'un élément d'insertion est construite dans un réseau 3D de 10 bloc, 10 lignes et 10 fonds de colonne contenant chacun l'ADN de 100 installations **caractérisée par** les trois coordonnées B, R, C.

10. Méthode selon l'une quelconque des revendications 1 à 4 où Bfal est utilisée sous forme d'enzyme de restriction.

11. Méthode selon la revendication 5 où Msel et/ou Munl sont utilisées sous forme d'enzyme de restriction.
